# EUROPEAN PATENT APPLICATION

(11) **EP 3 318 194 A1**
(43) Date of publication of application: **09.05.2018**
(21) Application number: 16817527.1
(22) Date of filing: 30.03.2016
(51) Int. Cl.: A61B 8/12

(54) **ULTRASONIC OBSERVATION APPARATUS, ULTRASONIC OBSERVATION APPARATUS OPERATION METHOD, AND ULTRASONIC OBSERVATION APPARATUS OPERATION PROGRAM**

(30) Priority: 02.07.2015 JP 2015133871
(71) Applicant: OLYMPUS CORPORATION, Hachioji-shi Tokyo 192-8507 (JP)
(72) Inventor: MIYAKE, Tatsuya, Tokyo 192-8507 (JP)
(74) Representative: Gunzelmann, Rainer
(86) International application number: PCT/JP2016/060573
(87) International publication number: WO 2017/002417

(57) **Abstract**

A puncture needle detection unit that detects an image of a puncture needle displayed in an ultrasound image, a motion extraction unit that extracts a linear motion at a point of the puncture needle based on a history of the image of the puncture needle in the ultrasound image, and a composite image generation unit that generates a composite image by generating a locus of the linear motion extracted by the motion extraction unit and superimposing the locus on the ultrasound image are included. With the configuration, an ultrasound observation apparatus, a method of operating an ultrasound observation apparatus, and an ultrasound observation apparatus operation program, which enable a user to grasp a position where the puncture needle has been moved a plurality of times in a subject, are provided.

## Description

### Field

The present invention relates to an ultrasound observation apparatus for observing a tissue to be observed, using ultrasound waves, a method of operating an ultrasound observation apparatus, and an ultrasound observation apparatus operation program.

### Background

Needle biopsy, which is performed using a puncture needle when diagnosing a tissue to be observed using ultrasound waves, has a very narrow range of tissues that can be collected by one puncture motion and has a small amount of tissues that can be collected. To collect a wide range of tissues, the needle biopsy may be performed a plurality of times at different positions on the same cross section. Further, to increase the collection amount of the tissues, the puncture needle may be reciprocated a plurality of times at the same position.

When performing the needle biopsy a plurality of times, it is not easy to grasp the position where the needle biopsy has been performed after the puncture needle has been taken out from the tissue to be observed. To solve this problem, conventionally, a technology of storing a position that the puncture needle has reached within a tissue and displaying the position on a monitor is disclosed (see, for example, Patent Literature 1).

### Citation List

### Patent Literature

Patent Literature 1: JP 2009-189500 A

### Summary

### Technical Problem

However, in the technology described in Patent Literature 1, motion of the puncture needle a plurality of times at the same position in a subject is not assumed. Therefore, the technology described in Patent Literature 1 cannot accurately grasp the position where the puncture needle has been moved a plurality of times in the subject.

The present invention has been made in view of the foregoing and an object of the present invention is to provide an ultrasound observation apparatus, a method of operating an ultrasound observation apparatus, and an ultrasound observation apparatus operation program, which enable a user to accurately grasp a position where a puncture needle has been moved a plurality of times in a subject.

### Solution to Problem

To solve the above-described problem and achieve the object, an ultrasound observation apparatus according to the present invention generates an ultrasound image based on an ultrasound echo acquired by an ultrasound probe provided with an ultrasound transducer that transmits an ultrasound wave to an observation target and receives an ultrasound wave reflected at the observation target, and includes: a puncture needle detection unit configured to detect an image of a puncture needle displayed in the ultrasound image; a motion extraction unit configured to extract a linear motion at a point of the puncture needle based on a history of the image of the puncture needle in the ultrasound image; and a composite image generation unit configured to generate a composite image by generating a locus of the linear motion extracted by the motion extraction unit and superimposing the locus on the ultrasound image.

The above-described ultrasound observation apparatus according to the present invention further includes: a puncture needle information storage unit configured to store information of a point position of the image of the puncture needle detected by the puncture needle detection unit, wherein the motion extraction unit extracts the linear motion by acquiring a history of the point position of the image of the puncture needle from the puncture needle information storage unit.

In the above-described ultrasound observation apparatus according to the present invention, the composite image generation unit generates loci of the linear motions having passed through a common section a plurality of times.

In the above-described ultrasound observation apparatus according to the present invention, the composite image generation unit changes a display form of the loci of the linear motions in the composite image according to number of times of passage in the common section.

In the above-described ultrasound observation apparatus according to the present invention, the composite image generation unit generates the composite image in which information of number of times of passage in the common section is added and displayed to the loci of the corresponding linear motions.

In the above-described ultrasound observation apparatus according to the present invention, the composite image generation unit superimposes, on the ultrasound image, a region indicating a range of the linear motions performed during time from when the puncture needle detection unit starts detecting the image of the puncture needle to when the puncture needle detection unit stops detecting the image of the puncture needle.

In the above-described ultrasound observation apparatus according to the present invention, the composite image generation unit generates the composite image while holding a relative positional relationship between the observation target displayed in the ultrasound image and the locus of the puncture needle.

In the above-described ultrasound observation apparatus according to the present invention, the composite image generation unit starts generation of the composite image when the puncture needle detection unit stops detecting the image of the puncture needle after a state in which the puncture needle detection unit is detecting the image of the puncture needle is continued.

In the above-described ultrasound observation apparatus according to the present invention, the composite image generation unit stops generation of the composite image when the puncture needle detection unit detects the image of the puncture needle in a case where the composite image generation unit has generated the composite image.

In the above-described ultrasound observation apparatus according to the present invention, the composite image generation unit generates a composite image displaying loci of the linear motions during time from when the puncture needle detection unit detects the image of the puncture needle to when the puncture needle detection unit stops detecting the image of the puncture needle, and loci of the linear motions during time from when the puncture needle detection unit detects the image of the puncture needle again to when the puncture needle detection unit stops detecting the image of the puncture needle, in different display forms from each other.

In the above-described ultrasound observation apparatus according to the present invention, the composite image generation unit generates the composite image during when the puncture needle detection unit is detecting the image of the puncture needle.

A method of operating an ultrasound observation apparatus according to the present invention is a method of operating an ultrasound observation apparatus for generating an ultrasound image based on an ultrasound echo acquired by an ultrasound probe provided with an ultrasound transducer that transmits an ultrasound wave to an observation target and receives an ultrasound wave reflected at the observation target, and includes: a puncture needle detection step of detecting, by a puncture needle detection unit, an image of a puncture needle displayed in the ultrasound image; a motion extraction step of extracting, by a motion extraction unit, a linear motion at a point of the puncture needle based on a history of the image of the puncture needle in the ultrasound image; and a composite image generation step of generating, by a composite image generation unit, a composite image by generating a locus of the linear motion extracted by the step of extracting and superimposing the locus on the ultrasound image.

An ultrasound observation apparatus operation program according to the present invention causes an ultrasound observation apparatus for generating an ultrasound image based on an ultrasound echo acquired by an ultrasound probe provided with an ultrasound transducer that transmits an ultrasound wave to an observation target and receives an ultrasound wave reflected at the observation target, to execute: a puncture needle detection step of detecting, by a puncture needle detection unit, an image of a puncture needle displayed in the ultrasound image; a motion extraction step of extracting, by a motion extraction unit, a linear motion at a point of the puncture needle based on a history of the image of the puncture needle in the ultrasound image; and a composite image generation step of generating, by a composite image generation unit, a composite image by generating a locus of the linear motion extracted by the step of extracting and superimposing the locus on the ultrasound image. Advantageous Effects of Invention

According to the present invention, a composite image is generated by extracting a linear motion at a point of the puncture needle based on a history of an image of the puncture needle in an ultrasound image, and generating a locus of the linear motion and superimposing the locus on the ultrasound image. Therefore, the position where the puncture needle has been moved a plurality of times in a subject can be accurately grasped.

### Brief Description of Drawings

FIG. 1 is a diagram schematically illustrating a configuration of an ultrasound diagnosis system including an ultrasound observation apparatus according to a first embodiment of the present invention.
FIG. 2 is a perspective view schematically illustrating a configuration of a distal end portion of an insertion portion and a distal end of a rigid portion of an ultrasound endoscope.
FIG. 3 is a block diagram illustrating functional configurations of the ultrasound observation apparatus according to the first embodiment of the present invention and devices connected to the ultrasound observation apparatus.
FIG. 4 is a diagram schematically illustrating a state in which an image of a puncture needle is displayed on a B-mode image.
FIG. 5 is a diagram schematically illustrating a display example of a composite image generated by a composite image generation unit of the ultrasound observation apparatus according to the first embodiment of the present invention.
FIG. 6 is a flowchart illustrating an outline of processing performed by the ultrasound observation apparatus according to the first embodiment of the present invention.
FIG. 7 is a diagram schematically illustrating a display example of a composite image in a first modification of the first embodiment of the present invention.
FIG. 8 is a diagram schematically illustrating a display example of a composite image in a second modification of the first embodiment of the present invention.
FIG. 9 is a diagram schematically illustrating a display example of a composite image in a third modification of the first embodiment of the present invention.
FIG. 10 is a flowchart illustrating an outline of processing performed by an ultrasound observation apparatus according to a second embodiment of the present invention.
FIG. 11 is a diagram schematically illustrating a display example of a composite image according to the second embodiment of the present invention.

### Description of Embodiments

Hereinafter, modes for carrying out the present invention (hereinafter referred to as "embodiments") will be described with reference to the accompanying drawings.

### (First Embodiment)

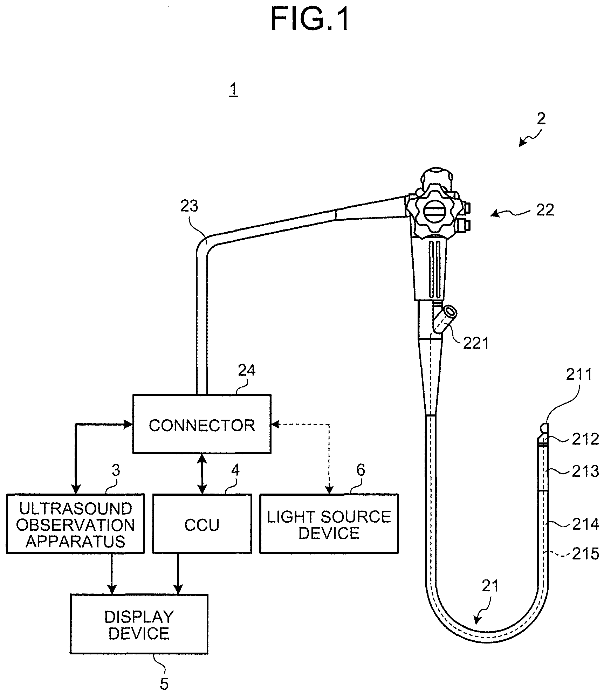

FIG. 1 is a diagram schematically illustrating a configuration of an ultrasound diagnosis system including an ultrasound observation apparatus according to a first embodiment of the present invention. An ultrasound diagnosis system 1 illustrated in FIG. 1 includes an ultrasound endoscope 2, an ultrasound observation apparatus 3, a camera control unit (CCU) 4, a display device 5, and a light source device 6.

The ultrasound endoscope 2 transmits ultrasound waves to a subject that is an observation target and receives the ultrasound waves reflected at the subject. The ultrasound endoscope 2 includes a tubular insertion portion 21 that is inserted into the subject, an operating unit 22 provided to a proximal end portion of the insertion portion 21 and which is held by a user and receives an operation input from the user, an universal cord 23 extending from the operating unit 22 and including a plurality of signal cables, an optical fiber that transmits illumination light generated by the light source device 6, and the like, and a connector 24 provided on an end portion of the universal cord 23 on an opposite side of the operating unit 22. For example, the ultrasound endoscope 2 observes digestive tract (esophagus, stomach, duodenum, or large intestine) and respiratory tract (trachea or bronchus) of the subject, as the observation target, and various types are known depending on the observation target.

The insertion portion 21 includes a distal end portion 211 in which an ultrasound transducer is provided, a rigid portion 212 externally covered with a rigid member connected to a proximal end side of the distal end portion 211, a bend portion 213 provided to a proximal end side of the rigid portion 212 and bendable according to the operation input received by the operating unit 22, and a flexible tube portion 214 provided to a proximal end side of the bend portion 213 and externally covered with a member having flexibility. A treatment tool channel 215 that is an insertion passage into which a treatment tool including a puncture needle is inserted is formed inside the insertion portion 21 (the treatment instrument channel is illustrated by the broken line in FIG. 1). Further, a light guide that transmits the illumination light supplied from the light source device 6 and a plurality of signal cables that transmits various signals are provided inside the insertion portion 21 (not illustrated).

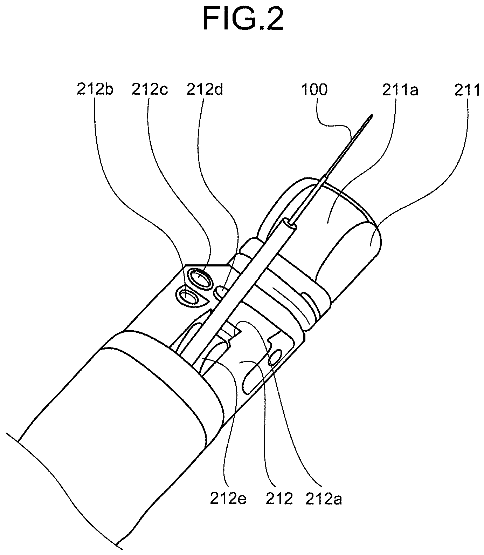

FIG. 2 is a perspective view schematically illustrating a configuration of the distal end portion 211 of the insertion portion 21 and a distal end of the rigid portion 212. The distal end portion 211 includes a convex ultrasound transducer 211a. The ultrasound transducer 211a may be an electronic scanning-type transducer or a mechanical scanning-type transducer. A treatment tool opening portion 212a communicating with the treatment tool channel 215, an imaging opening portion 212b collecting light from an outside and guiding the light to an imaging optical system, an illumination opening portion 212c located at a distal end side of the light guide and which emits the illumination light, and an air and water feed nozzle 212d are provided in a distal end of the rigid portion 212. The treatment tool opening portion 212a is provided with a rising base 212e that allows the treatment tool to be placed thereon in a manner that a protruding direction of the instrument tool to an outside is changeable. The rising base 212e is capable of changing a rising angle by an operation input of the operating unit 22. An objective lens is attached to the imaging opening portion 212b and an illumination lens is attached to the illumination opening portion 212c.

FIG. 2 illustrates a state in which a puncture needle 100, which is a kind of the treatment tool, protrudes from the treatment tool opening portion 212a. The puncture needle 100 is inserted into the treatment tool channel 215 via a treatment tool insertion port 221 (see FIG. 1) formed in the operating unit 22, and protrudes to an outside through the treatment tool opening portion 212a.

The configuration of the ultrasound diagnosis system 1 is continuously described with reference to FIG. 1. The connector 24 connects the ultrasound observation apparatus 3, the camera control unit 4, and the light source device 6. From the connector 24, an ultrasound cable that transmits and receives a signal to and from the ultrasound observation apparatus 3, an electrical cable that transmits and receives a signal to and from the camera control unit 4, and the light guide that transmits the illumination light generated by the light source device 6 extend. An ultrasound connector connected to the ultrasound observation apparatus 3 is provided in a distal end of the ultrasound cable. An electrical connector connected to the camera control unit 4 is provided in a distal end of the electrical cable.

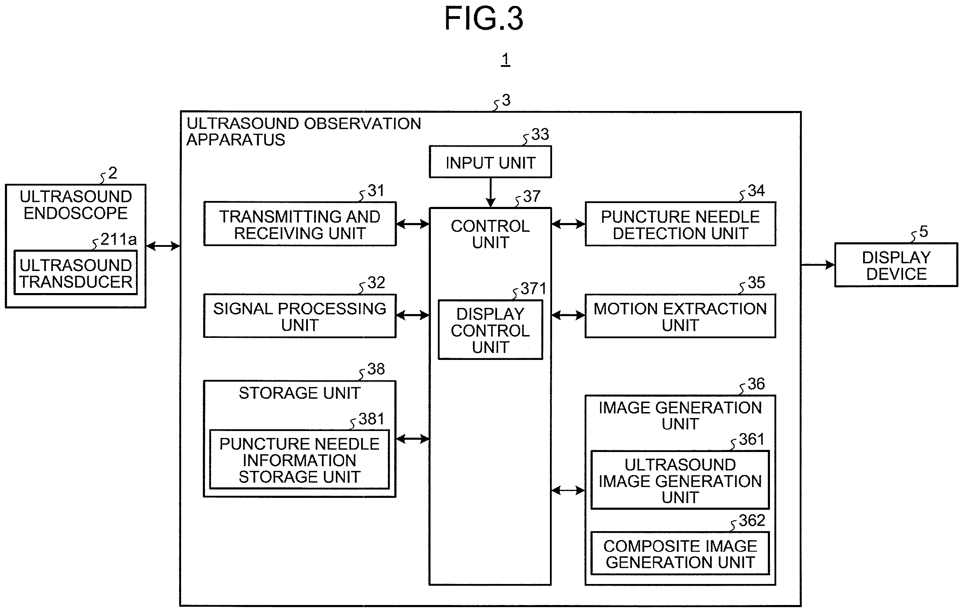

The ultrasound observation apparatus 3 transmits and receives an electrical signal to and from the ultrasound endoscope 2 via the ultrasound cable. The ultrasound observation apparatus 3 applies predetermined processing to an electrical echo signal received from the ultrasound endoscope 2 to generate an ultrasound image or the like. Details of the function and configuration of the ultrasound observation apparatus 3 will be described below with reference to the block diagram of FIG. 3.

The camera control unit 4 applies predetermined processing to an image signal received from the ultrasound endoscope 2 through the electrical cable to generate an endoscope image.

The display device 5 is configured using liquid crystal, organic electro luminescence (EL), or the like, and receives data of the ultrasound image generated by the ultrasound observation apparatus 3, the endoscope image generated by the camera control unit 4, and the like and displays the images.

The light source device 6 generates the illumination light for illuminating an inside of the subject and supplies the illumination light to the ultrasound endoscope 2 via the light guide. The light source device 6 also incorporates a pump for sending water and air.

FIG. 3 is a block diagram illustrating functional configurations of the ultrasound observation apparatus according to the first embodiment and devices connected to the ultrasound observation apparatus. The ultrasound observation apparatus 3 includes a transmitting and receiving unit 31 that transmits and receives a signal to and from the ultrasound transducer 211a, a signal processing unit 32 that generates digital reception data based on an echo signal received from the transmitting and receiving unit 31, an input unit 33 realized using a user interface of a keyboard, a mouse, and a touch panel, and which receives inputs of various types of information including a motion instruction signal of the ultrasound observation apparatus 3, a puncture needle detection unit 34 that detects the puncture needle included in the ultrasound image, a motion extraction unit 35 that extracts a linear motion of the puncture needle based on a history of a position of a point of an image of the puncture needle in the ultrasound image, an image generation unit 36 that generates data of various types of images including the ultrasound image, using information of the reception data generated by the signal processing unit 32, a control unit 37 that collectively controls the operation of the entire ultrasound diagnosis system 1, and a storage unit 38 that stores various types of information necessary for the operation of the ultrasound observation apparatus 3.

The transmitting and receiving unit 31 transmits a pulse transmission drive wave signal to the ultrasound transducer 211a based on a predetermined waveform and transmission timing. Further, the transmitting and receiving unit 31 receives an electrical echo signal from the ultrasound transducer 211a. The transmitting and receiving unit 31 also has functions to transmit various control signals outputted by the control unit 37 to the ultrasound endoscope 2, and receive various types of information including an ID for identification from the ultrasound endoscope 2 and transmit the information to the control unit 37.

The signal processing unit 32 applies known processing such as band-pass filter, envelope detection, and logarithmic conversion to the echo signal to generate digital ultrasound image reception data, and outputs the generated data. The signal processing unit 32 is realized using a general-purpose processor such as a central processing unit (CPU) or a dedicated integrated circuit or the like that executes a specific function such as application specific integrated circuit (ASIC) or field programmable gate array (FPGA).

The puncture needle detection unit 34 detects the puncture needle displayed in the ultrasound image by image processing, and writes and stores coordinates of the position of the point of the detected puncture needle in the ultrasound image together with information of detection time to a puncture needle information storage unit 381 included in the storage unit 38. The puncture needle detection unit 34 detects, for example, a region having a large luminance value as the puncture needle by analyzing the luminance of pixels of the ultrasound image. Note that the puncture needle detection unit 34 may detect the puncture needle by performing pattern matching using the ultrasound image of the puncture needle stored in advance by the storage unit 38.

The motion extraction unit 35 extracts a motion that forms a linear locus in the ultrasound image based on the puncture needle information stored in the puncture needle information storage unit 381. At this time, the motion extraction unit 35 extracts movement of the puncture needle from the start to the end in the same direction as a single motion. By extracting the motion that forms a linear locus by the motion extraction unit 35 in this way, a locus of when changing a direction to puncture by the puncture needle 100 by changing the rising angle of the rising base 212e is deleted, for example. Note that "linear" referred to here includes not only the case where the point of the puncture needle is moved on one straight line in the ultrasound image but also a case where the point of the puncture needle is moved in a long and narrow rectangular region having a small width set in advance in a direction orthogonal to a moving direction along the straight line.

The image generation unit 36 includes an ultrasound image generation unit 361 that generates ultrasound image data based on the reception data, and a composite image generation unit 362 that generates a composite image by generating the locus of the linear motion of the puncture needle extracted by the motion extraction unit 35 and superimposing the locus on the ultrasound image.

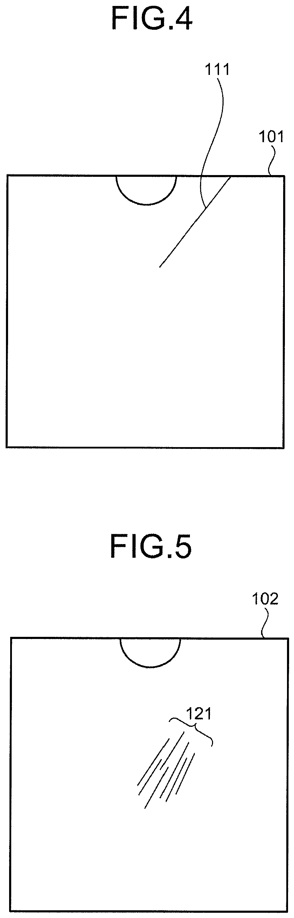

The ultrasound image generated by the ultrasound image generation unit 361 is a B-mode image obtained by converting amplitude into luminance. FIG. 4 is a diagram schematically illustrating a state in which an image of a puncture needle is displayed in a B-mode image. In a B mode image 101 illustrated in FIG. 4, a puncture needle image 111 linearly extending from an upper right part toward a central part of a screen is displayed. Note that, in FIG. 4, concrete display of the ultrasound image other than the puncture needle image 111 is omitted. The omission of concrete image of the ultrasound image other than the puncture needle image 111 is similarly applied to the composite image to be referred to below.

The composite image generation unit 362 generates a composite image by superimposing the locus of the linear motion extracted by the motion extraction unit 35 on the ultrasound image. FIG. 5 is a diagram schematically illustrating a display example of a composite image generated by the composite image generation unit 362. In a composite image 102 illustrated in FIG. 5, a locus group 121 composed of a plurality of straight lines is displayed. In the first embodiment, the composite image generation unit 362 generates a composite image after the puncture needle image becomes undetected for the first time after the puncture needle detection unit 34 starts detecting the image of the puncture needle. That is, in the first embodiment, the composite image generation unit 362 generates the composite image after the puncture needle detection unit 34 stops detecting the image of the puncture needle due to taking out of the puncture needle from the ultrasound endoscope 2 after termination of the first needle biopsy.

The composite image generation unit 362 generates the composite image by arranging lines representing the locus of the linear motion extracted by the motion extraction unit 35 while adjusting a display area of the image of the puncture needle in accordance with change of the position of the observation target, of each frame, displayed in the B-mode image.

In a case where the puncture needle detection unit 34 again detects the image of the puncture needle after the composite image generation unit 362 generates the composite image, the composite image generation unit 362 stops generation of the composite image. In this case, the B-mode image is displayed on the display device 5. In this case, the composite image generation unit 362 may generate a composite image displaying a locus in a form not disturbing the visibility of the puncture needle. Examples of the form not disturbing the visibility of the puncture needle include displaying the locus by a broken line and displaying the locus in a less visible color.

In a case where a freeze button of the operating unit 22 receives an operation input after the composite image generation unit 362 generates the composite image, the composite image generation unit 362 may stop generation of the composite image. In this case, when canceling the freeze upon receipt of an operation input again by the freeze button, the composite image generation unit 362 may resume the generation of the composite image.

The control unit 37 includes a display controller 371 that controls display of the display device 5. The display controller 371 causes the display device 5 to display the various images generated by the image generation unit 36.

The control unit 37 is realized using a general-purpose processor such as a CPU having arithmetic and control functions, or a dedicated integrated circuit such as ASIC or FPGA. In the case where the control unit 37 is realized by the general-purpose processor or the FPGA, the control unit 37 reads various programs and data stored in the storage unit 38 from the storage unit 38, and executes various types of arithmetic processing related to the operation of the ultrasound observation apparatus 3 to collectively control the ultrasound observation apparatus 3. In the case where the control unit 37 is configured from the ASIC, the control unit 37 may independently execute various types of processing, or may execute the various types of processing using various data stored in the storage unit 38. In the first embodiment, the control unit 37 and part of the signal processing unit 32, the puncture needle detection unit 34, the motion extraction unit 35, and the image generation unit 36 can be configured from a common general-purpose processor, a dedicated integrated circuit, or the like.

The storage unit 38 includes the puncture needle information storage unit 381 that stores information of the point position of the image of the puncture needle detected by the puncture needle detection unit 34 together with the information of the detection time of the image of the puncture needle and the like, as puncture needle information. The puncture needle information stored in the puncture needle information storage unit 381 is deleted under control of the control unit 37 when the puncture needle detection unit 34 starts detecting the image of the puncture needle again after having stopped detecting the image of the puncture needle.

The storage unit 38 stores various programs including an operation program for executing an operation method of the ultrasound observation apparatus 3. The various programs including an operation program can also be recorded on a computer readable recording medium such as a hard disk, a flash memory, a CD-ROM, a DVD-ROM, or a flexible disk and widely distributed. Note that the above-described various programs can also be acquired by being downloaded via a communication network. The communication network referred to here is realized by, for example, an existing public line network, a local area network (LAN), or a wide area network (WAN), and may be a wired or wireless network.

The storage unit 38 is realized using a read only memory (ROM) in which the various programs and the like are installed in advance, a random access memory (RAM) in which arithmetic parameters and data of processing, and the like are stored, and the like.

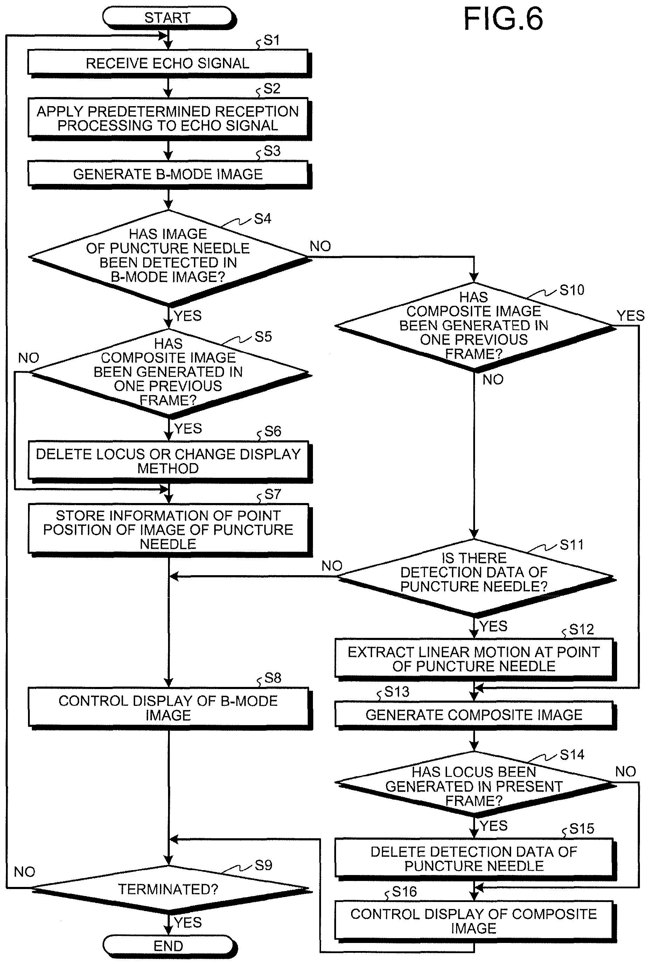

FIG. 6 is a flowchart illustrating an outline of processing performed by the ultrasound observation apparatus 3. The flowchart illustrated in FIG. 6 illustrates processing after the transmitting and receiving unit 31 starts transmission of a transmission drive wave according to an observation mode, and the ultrasound transducer 211a starts transmission of an ultrasound wave.

First, the transmitting and receiving unit 31 receives an echo signal that is a measurement result of an observation target by the ultrasound transducer 211a from the ultrasound endoscope 2 (Step S1).

Next, the transmitting and receiving unit 31 applies predetermined reception processing to the echo signal received from the ultrasound transducer 211a (Step S2). To be specific, the transmitting and receiving unit 31 amplifies (STC correction) the echo signal and then applies processing such as filtering or A/D conversion to the amplified signal.

After that, the ultrasound image generation unit 361 generates a B-mode image, using the echo signal processed by the transmitting and receiving unit 31, and outputs data of the B-mode image to the display device 5 (Step S3).

The puncture needle detection unit 34 performs processing of detecting the image of the puncture needle displayed in the B-mode image, using the generated B-mode image (Step S4). When the puncture needle detection unit 34 detects the image of the puncture needle in the B mode image (Step S4: Yes), and when the composite image generation unit 362 has generated a composite image in a previous frame (Step S5: Yes), the ultrasound observation apparatus 3 proceeds to Step S6.

In Step S6, the display controller 371 deletes (non-displays) the locus or changes the display method (Step S6). The puncture needle detection unit 34 writes and stores the information of the point position of the image of the detected puncture needle together with the information of detection time and the like to the puncture needle information storage unit 381 (Step S7). The information of the point position of the image of the puncture needle is represented by coordinates in the B-mode image, for example. Step S6 corresponds to a situation in which the puncture needle is newly inserted while the display device 5 is displaying the composite image. In Step S6, the locus of the previous puncture needle may be deleted, or the display method may be changed so that the locus can be identified as the previous locus.

Next, the display controller 371 performs control to cause the display device 5 to display the B-mode image (Step S8).

After that, when the input unit 33 receives an input of a signal instructing termination (Step S9: Yes), the ultrasound observation apparatus 3 terminates the series of processing. On the other hand, when the input unit 33 does not receive the input of a signal instructing termination (Step S9: No), the ultrasound observation apparatus 3 returns to Step S1.

When the puncture needle detection unit 34 detects the image of the puncture needle in the B-mode image (Step S4: Yes), and when the composite image generation unit 362 has not generated the composite image in the previous frame (Step S5: No), the ultrasound observation apparatus 3 proceeds to Step S7.

The case in which the puncture needle detection unit 34 does not detect the image of the puncture needle in the B-mode image (Step S4: No) in Step S4 will be described. In this case, when the composite image generation unit 362 has not generated the composite image in the previous frame (Step S10: No), the ultrasound observation apparatus 3 proceeds to Step S11. This situation corresponds to a situation in which the puncture needle detection unit 34 has never detected the puncture needle, or a situation in which the puncture needle detection unit 34 has continued to detect the image of the puncture needle up to one previous frame and stops detecting the image of the puncture needle at this frame.

In Step S11, when the puncture needle information storage unit 381 stores detection data of the puncture needle (Step S11: Yes), that is, when the puncture needle information storage unit 381 has stored the information of the point position of the image of the puncture needle in a plurality of frames up to the previous frame in succession, the motion extraction unit 35 extracts the linear motion at the point of the puncture needle based on the history of the point position of the image of the puncture needle (Step S12).

In Step S11, when the puncture needle information storage unit 381 does not store the detection data of the puncture needle (Step S11: No), the ultrasound observation apparatus 3 proceeds to Step S8 described above. This situation corresponds to a situation in which the puncture needle detection unit 34 has never detected the puncture needle.

After that, the composite image generation unit 362 generates the locus of the linear motion extracted by the motion extraction unit 35, and superimposes the locus on the B-mode image to generate a composite image (Step S13). At this time, the composite image generation unit 362 generates the composite image by performing correction to hold a relative positional relationship between the locus of the linear motion at the point of the puncture needle and the observation target of the B-mode image.

When the composite image generation unit 362 generates the locus in this frame (Step S14: Yes), the control unit 37 deletes the detection data of the puncture needle stored in the puncture needle information storage unit 381 (Step S15).

Next, the display controller 371 performs control to cause the display device 5 to display the composite image generated by the composite image generation unit 362 (Step S16). The composite image displayed by the display device 5 is, for example, the composite image 102 illustrated in FIG. 5.

After Step S16, the ultrasound observation apparatus 3 proceeds to Step S9 described above.

In Step S10, when the composite image generation unit 362 has generated the composite image in the previous frame (Step S10: Yes), the ultrasound observation apparatus 3 proceeds to Step S13. This situation corresponds to a situation in which undetected state of the image of the puncture needle continues from at least one previous frame. In this situation, in Step S13, the composite image generation unit 362 generates the composite image, using the newly generated B-mode image and the locus generated in one previous frame.

In Step S14, when the composite image generation unit 362 has not generated the locus in this frame (Step S14: No), that is, when the composite image generation unit 362 has generated the locus in a frame prior to the present frame, the ultrasound observation apparatus 3 proceeds to Step S16.

According to the first embodiment of the present invention described above, the composite image is generated by extracting the linear motion at the point of the puncture needle based on the history of the image of the puncture needle in the ultrasound image, and generating the locus of the extracted linear motion and superimposing the locus on the ultrasound image. Therefore, the position where the puncture needle has been moved a plurality of times in the subject can be accurately grasped.

In the first embodiment, when the puncture needle detection unit 34 stops detecting the image of the puncture needle, the composite image generation unit 362 starts generation of the composite image, and after that, when the puncture needle detection unit 34 detects the image of the puncture needle again, the composite image generation unit 362 stops generation of the composite image. Therefore, according to the first embodiment, the user can confirm the history of the first needle biopsy during time from when the puncture needle is taken out once to when the second needle biopsy is performed, and can more accurately grasp the position where a tissue is to be collected in the second needle biopsy.

### (First Modification)

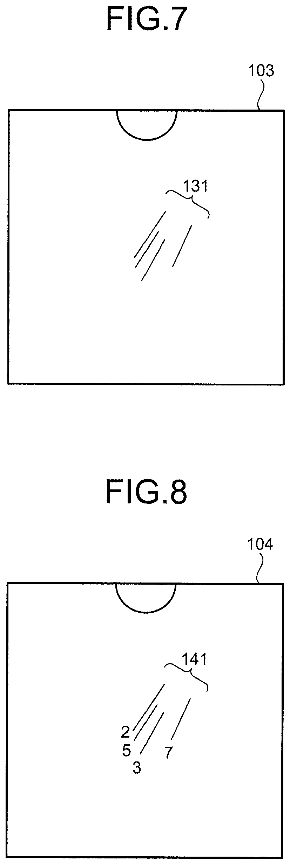

FIG. 7 is a diagram schematically illustrating a display example of a composite image in a first modification of the first embodiment. In the first modification, the composite image generation unit 362 generates a composite image, using only loci that have passed through a common section a plurality of times, of linear motions extracted by the motion extraction unit 35. A composite image 103 illustrated in FIG. 7 is an image generated based on the same puncture needle information as the composite image 102 illustrated in FIG. 5. A locus group 131 displayed in the composite image 103 illustrates only the loci that have passed through a common section a plurality of times. Therefore, the number of loci is smaller than that of the locus group 121 displayed on the composite image 102 generated based on the same puncture needle information.

According to the first modification described above, only the loci including an overlapping portion in a plurality of motions are displayed. Therefore, a portion having a high possibility of collecting a tissue can be displayed. Therefore, a user can more reliably specify a place having a high possibility of collecting a tissue.

Note that, in the first modification, loci up to a predetermined place in descending order of the number of times of overlap may be displayed.

### (Second Modification)

FIG. 8 is a diagram schematically illustrating a display example of a composite image in a second modification of the first embodiment. In the second modification, a composite image generation unit 362 adds the numbers of times of overlap near loci, in addition to generation of a composite image, using only the loci having passed through a common section a plurality of times, of linear motions extracted by a motion extraction unit 35, similarly to the first modification. The composite image 104 illustrated in FIG. 8 is an image generated based on the same puncture needle information as the composite image 102 illustrated in FIG. 5. A locus group 141 displayed in the composite image 104 displays the same loci as the locus group 131 of the composite image 103 and the number of times of overlap is displayed near each locus.

According to the second modification described above, only the loci including an overlapping portion in a plurality of motions is displayed together with the numbers of times of overlap. Therefore, the user can more easily grasp a portion having a high possibility of collecting a tissue.

Note that, in the second modification, a composite image that displays colors and types of lines of the loci in different forms according to the number of times of overlap may be generated instead of displaying the number of times of overlap. Further, in the second modification, loci up to a predetermined place in descending order of the number of times of overlap may be displayed, similarly to the first modification.

### (Third Modification)

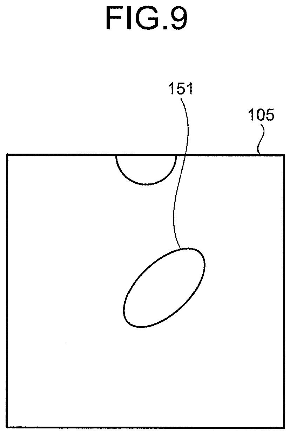

FIG. 9 is a diagram schematically illustrating a display example of a composite image in a third modification of the first embodiment. In the third modification, the composite image generation unit 362 generates a composite image by superimposing a region indicating a range where an image of a puncture needle has performed linear motions on a B-mode image during time from when the puncture needle detection unit 34 starts detecting the image of the puncture needle to when the puncture needle detection unit 34 stops detecting the image of the puncture needle. A composite image 105 illustrated in FIG. 9 is an image generated based on the same puncture needle information as the composite image 102 illustrated in FIG. 5. An approximately elliptical region 151 illustrated in the composite image 105 is the region indicating a range of linear motions of the puncture needle. For example, the region 151 may be set as an outermost contour of all the linear loci or may be set as an envelope surrounding all the linear loci.

According to the third modification, in a case where a user wants to puncture another region in the next biopsy motion, the user can easily grasp the region to be newly punctured.

Note that, in a case where a puncture needle detection unit 34 detects an image of a puncture needle again after a composite image generation unit 362 generates a composite image displaying loci, as described in the first embodiment and first and second modifications, the composite image generation unit 362 may start generation of a composite image with contour display described in the third modification. In this case, when the composite image generation unit 362 resumes generation of the composite image displaying loci when the puncture needle detection unit 34 stops detecting the image of the puncture needle. The composite image generation unit 362 may start generation of the composite image with contour display when receiving an operation input of a freeze button of an operating unit 22, and may resume generation of the composite image displaying loci when receiving a re-operation input of the freeze button.

### (Second Embodiment)

A second embodiment of the present invention is characterized in displaying a locus of an image of a puncture needle in an ultrasound image substantially in real time. A configuration of an ultrasound observation apparatus according to the second embodiment is similar to that of the ultrasound observation apparatus 3 described in the first embodiment.

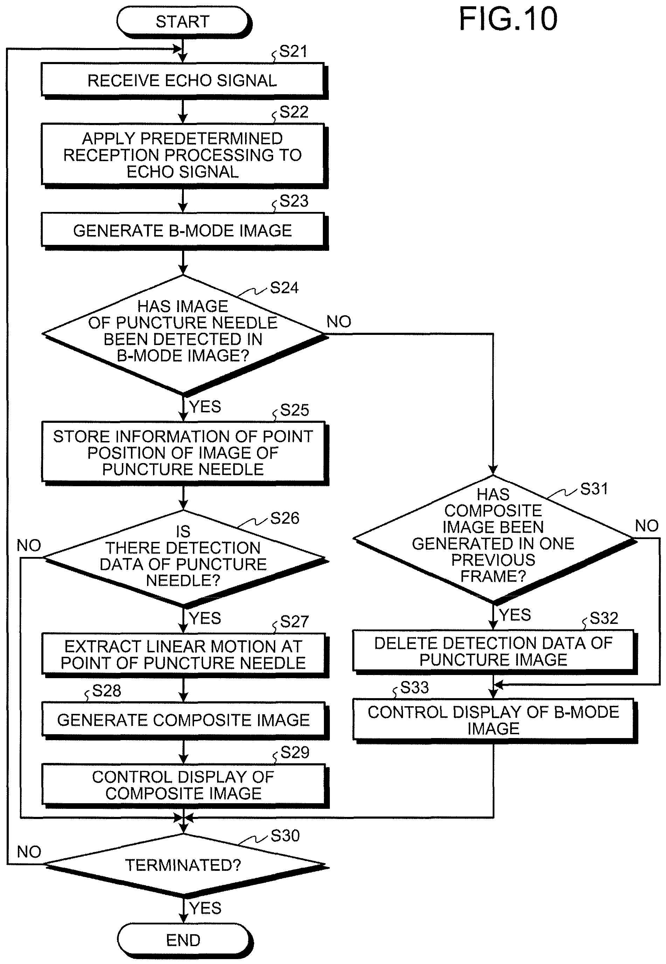

FIG. 10 is a flowchart illustrating an outline of processing performed by an ultrasound observation apparatus 3 according to the second embodiment. Processing of Steps S21 to S23 sequentially corresponds to the processing of Steps S1 to S3 described in the first embodiment.

Following Step S23, a puncture needle detection unit 34 performs processing of detecting an image of a puncture needle displayed in a B-mode image using the generated B-mode image (Step S24). When the puncture needle detection unit 34 detects the image of the puncture needle in the B-mode image (Step S24: Yes), the puncture needle detection unit 34 writes and stores information of a point position of the image of the detected puncture needle together with information of detection time and the like to a puncture needle information storage unit 381 (Step S25).

After that, when the puncture needle information storage unit 381 stores detection data of the puncture needle (Step S26: Yes), that is, when the puncture needle information storage unit 381 has stored the image of the point position of the image of the puncture needle in a plurality of frames up to a previous frame in succession, a motion extraction unit 35 extracts a linear motion at the point of the puncture needle based on a history of the point position of the image of the puncture needle (Step S27). Meanwhile, when the puncture needle information storage unit 381 does not store the puncture needle information (Step S26: No), the ultrasound observation apparatus 3 proceeds to Step S30 described below.

After Step S27, a composite image generation unit 362 generates a composite image by superimposing the locus of the linear motion extracted by the motion extraction unit 35 on an ultrasound image (Step S28).

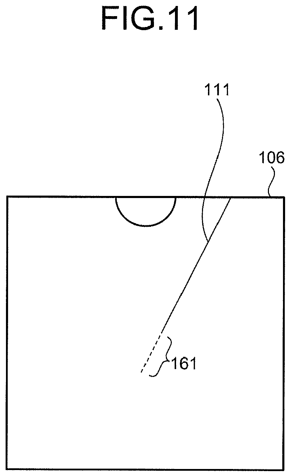

Next, a display controller 371 performs control to cause a display device 5 to display the composite image generated by the composite image generation unit 362 (Step S29). FIG. 11 is a diagram schematically illustrating a display example of a composite image displayed by the display device 5. A composite image 106 illustrated in FIG. 11 is displayed in a manner that a puncture needle image 111 and a locus 161 of a point position of the puncture needle image 111 can be identified. FIG. 11 illustrates a case where the locus 161 is displayed by a broken line. However, the locus 161 may be displayed in a color different from the puncture needle image 111 or may be displayed with a different thickness from the puncture needle image 111.

After that, when an input unit 33 receives an input of a signal instructing termination (Step S30: Yes), the ultrasound observation apparatus 3 terminates the series of processing. On the other hand, when the input unit 33 does not receive the input of a signal instructing termination (Step S30: No), the ultrasound observation apparatus 3 returns to Step S21.

A case in which the puncture needle detection unit 34 does not detect the image of the puncture needle in the B-mode image (Step S24: No) will be described. In this case, when the ultrasound observation apparatus 3 generates the composite image in one previous frame (Step S31: Yes), the control unit 37 deletes detection data of the puncture needle stored in the puncture needle information storage unit 381 (Step S32).

After that, the display controller 371 performs control to cause the display device 5 to display the B-mode image generated in Step S23 (Step S33). As described above, in the second embodiment, the locus of the puncture needle is not displayed when the image of the puncture needle is not included in the B-mode image. After Step S33, the ultrasound observation apparatus 3 proceeds to Step S30.

In Step S31, when the ultrasound observation apparatus 3 has not generated a composite image in one previous frame (Step S31: No), the ultrasound observation apparatus 3 proceeds to Sep S33.

Note that the display controller 371 may cause the display device 5 to display the composite image in Step S33 without performing the processing of Step S32. In that case, when the puncture needle is newly detected, the display controller 371 deletes and non-displays the locus or changes the display method.

According to the second embodiment of the present invention described above, the composite image is generated by extracting the linear motion at the point of the puncture needle based on the history of the image of the puncture needle in the ultrasound image, and generating the locus of the extracted linear motion and superimposing the locus on the ultrasound image. Therefore, the position where the puncture needle has been moved a plurality of times in the subject can be accurately grasped.

Further, according to the second embodiment, the linear locus of the image of the puncture needle can be grasped substantially in real time. Therefore, a position where a tissue is to be collected next can be specified in one-time needle biopsy.

The embodiments for carrying out the present invention have been described. However, the present invention should not be limited only by the above-described first and second embodiments. For example, in the first and second embodiments, when the puncture needle detection unit 34 starts detecting the image of the puncture needle again after stopping detecting the image of the puncture needle, the puncture needle information storage unit 381 may continuously provide and store information to be newly stored and identifiable additional information to the information of the point position of the image of the puncture needle stored in the puncture needle information storage unit 381 so far. In this case, the composite image generation unit 362 may generate a composite image displaying loci of the puncture needle respectively corresponding to the old and new information, that is, loci of the puncture needle in different needle biopsies in an identifiable manner.

Further, as an ultrasound probe, an extracorporeal ultrasound probe that irradiates a body surface of a subject with ultrasound waves may be applied. The extracorporeal ultrasound probe is usually used for observing abdominal organs (liver, gall bladder, and bladder), breast (especially mammary gland), and thyroid gland.

As described above, the present invention may include various embodiments within the scope not deviating from the technical idea described in the claims.

### Reference Signs List

1 ULTRASOUND DIAGNOSIS SYSTEM
2 ULTRASOUND ENDOSCOPE
3 ULTRASOUND OBSERVATION APPARATUS
4 CAMERA CONTROL UNIT
5 DISPLAY DEVICE
6 LIGHT SOURCE DEVICE
21 INSERTION PORTION
31 TRANSMITTING AND RECEIVING UNIT
32 SIGNAL PROCESSING UNIT
33 INPUT UNIT
34 PUNCTURE NEEDLE DETECTION UNIT
35 OPERATION EXTRACTION UNIT
36 IMAGE GENERATION UNIT
37 CONTROL UNIT
38 STORAGE UNIT
100 PUNCTURE NEEDLE
101 B-MODE IMAGE
102, 103, 104, 105, 106 COMPOSITE IMAGE
111 PUNCTURE NEEDLE IMAGE
121, 131, 141 LOCUS GROUP
151 REGION
161 LOCUS
211 DISTAL END PORTION
211a ULTRASOUND TRANSDUCER
361 ULTRASOUND IMAGE GENERATION UNIT
362 COMPOSITE IMAGE GENERATION UNIT
371 DISPLAY CONTROLLER
381 PUNCTURE NEEDLE INFORMATION STORAGE UNIT

## Claims

1. An ultrasound observation apparatus for generating an ultrasound image based on an ultrasound echo acquired by an ultrasound probe provided with an ultrasound transducer that transmits an ultrasound wave to an observation target and receives an ultrasound wave reflected at the observation target, the ultrasound observation apparatus comprising:
a puncture needle detection unit configured to detect an image of a puncture needle displayed in the ultrasound image;
a motion extraction unit configured to extract a linear motion at a point of the puncture needle based on a history of the image of the puncture needle in the ultrasound image; and
a composite image generation unit configured to generate a composite image by generating a locus of the linear motion extracted by the motion extraction unit and superimposing the locus on the ultrasound image.

2. The ultrasound observation apparatus according to claim 1, further comprising:
a puncture needle information storage unit configured to store information of a point position of the image of the puncture needle detected by the puncture needle detection unit, wherein
the motion extraction unit extracts the linear motion by acquiring a history of the point position of the image of the puncture needle from the puncture needle information storage unit.

3. The ultrasound observation apparatus according to claim 1, wherein the composite image generation unit generates loci of the linear motions having passed through a common section a plurality of times.

4. The ultrasound observation apparatus according to claim 3, wherein the composite image generation unit changes a display form of the loci of the linear motions in the composite image according to number of times of passage in the common section.

5. The ultrasound observation apparatus according to claim 3, wherein the composite image generation unit generates the composite image in which information of number of times of passage in the common section is added and displayed to the loci of the corresponding linear motions.

6. The ultrasound observation apparatus according to claim 1, wherein the composite image generation unit superimposes, on the ultrasound image, a region indicating a range of the linear motions performed during time from when the puncture needle detection unit starts detecting the image of the puncture needle to when the puncture needle detection unit stops detecting the image of the puncture needle.

7. The ultrasound observation apparatus according to claim 1, wherein the composite image generation unit generates the composite image while holding a relative positional relationship between the observation target displayed in the ultrasound image and the locus of the puncture needle.

8. The ultrasound observation apparatus according to claim 1, wherein the composite image generation unit starts generation of the composite image when the puncture needle detection unit stops detecting the image of the puncture needle after a state in which the puncture needle detection unit is detecting the image of the puncture needle is continued.

9. The ultrasound observation apparatus according to claim 8, wherein the composite image generation unit stops generation of the composite image when the puncture needle detection unit detects the image of the puncture needle in a case where the composite image generation unit has generated the composite image.

10. The ultrasound observation apparatus according to claim 1, wherein the composite image generation unit generates a composite image displaying loci of the linear motions during time from when the puncture needle detection unit detects the image of the puncture needle to when the puncture needle detection unit stops detecting the image of the puncture needle, and loci of the linear motions during time from when the puncture needle detection unit detects the image of the puncture needle again to when the puncture needle detection unit stops detecting the image of the puncture needle, in different display forms from each other.

11. The ultrasound observation apparatus according to claim 1, wherein the composite image generation unit generates the composite image during when the puncture needle detection unit is detecting the image of the puncture needle.

12. A method of operating an ultrasound observation apparatus for generating an ultrasound image based on an ultrasound echo acquired by an ultrasound probe provided with an ultrasound transducer that transmits an ultrasound wave to an observation target and receives an ultrasound wave reflected at the observation target, the method comprising:
a puncture needle detection step of detecting, by a puncture needle detection unit, an image of a puncture needle displayed in the ultrasound image;
a motion extraction step of extracting, by a motion extraction unit, a linear motion at a point of the puncture needle based on a history of the image of the puncture needle in the ultrasound image; and
a composite image generation step of generating, by a composite image generation unit, a composite image by generating a locus of the linear motion extracted by the step of extracting and superimposing the locus on the ultrasound image.

13. An ultrasound observation apparatus operation program for causing an ultrasound observation apparatus for generating an ultrasound image based on an ultrasound echo acquired by an ultrasound probe provided with an ultrasound transducer that transmits an ultrasound wave to an observation target and receives an ultrasound wave reflected at the observation target, to execute:
a puncture needle detection step of detecting, by a puncture needle detection unit, an image of a puncture needle displayed in the ultrasound image;
a motion extraction step of extracting, by a motion extraction unit, a linear motion at a point of the puncture needle based on a history of the image of the puncture needle in the ultrasound image; and
a composite image generation step of generating, by a composite image generation unit, a composite image by generating a locus of the linear motion extracted by the step of extracting and superimposing the locus on the ultrasound image.
